# EUROPEAN PATENT APPLICATION

(11) **EP 1 783 493 A1**
(43) Date of publication of application: **09.05.2007**
(21) Application number: 06023147.9
(22) Date of filing: 07.11.2006
(51) Int. Cl.: G01N 33/543, G01N 33/548

(54) **Biosensor for detecting or measuring a substance that interacts with a physiologically active substance comprising a polysaccharide capable of chemically immobilizing physiologically active subtances**

(30) Priority: 08.11.2005 JP 2005323021; 08.11.2005 JP 2005323022
(71) Applicant: Fujifilm Corporation, Minato-Ku Tokyo 106-8620 (JP)
(72) Inventor: Kuruma, Koji, Kaisei-machi Ashigarakami-gun Kanagawa (JP); Ezoe, Toshihide, Kaisei-machi Ashigarakami-gun Kanagawa (JP)
(74) Representative: Grünecker, Kinkeldey, Stockmair & Schwanhäusser Anwaltssozietät

(57) **Abstract**

An object of the present invention is to provide a biosensor that is capable of immobilizing large quantities of physiologically active substances thereon. The present invention provides a biosensor comprising a substrate having on its surface a polysaccharide capable of chemically immobilizing physiologically active substances, wherein the polysaccharide is a linear polysaccharide or a polysaccharide having 0.3 to 2 primary alcohol groups per unit.

## Description

### Technical Field

The present invention relates to a biosensor and a method for analyzing an interaction between biomolecules using the biosensor. Particularly, the present invention relates to a biosensor which is used for a surface plasmon resonance biosensor and a method for analyzing an interaction between biomolecules using the biosensor.

### Background Art

Recently, a large number of measurements using intermolecular interactions such as immune responses are being carried out in clinical tests, etc. However, since conventional methods require complicated operations or labeling substances, several techniques are used that are capable of detecting the change in the binding amount of a test substance with high sensitivity without using such labeling substances. Examples of such a technique may include a surface plasmon resonance (SPR) measurement technique, a quartz crystal microbalance (QCM) measurement technique, and a measurement technique of using functional surfaces ranging from gold colloid particles to ultra-fine particles, The SPR measurement technique is a method of measuring changes in the refractive index near an organic functional film attached to the metal film of a chip by measuring a peak shift in the wavelength of reflected light, or changes in amounts of reflected light in a certain wavelength, so as to detect adsorption and desorption occurring near the surface. The QCM measurement technique is a technique of detecting adsorbed or desorbed mass at the ng level, using a change in frequency of a crystal due to adsorption or desorption of a substance on gold electrodes of a quartz crystal (device). In addition, the ultra-fine particle surface (nm level) of gold is functionalized, and physiologically active substances are immobilized thereon. Thus, a reaction to recognize specificity among physiologically active substances is carried out, thereby detecting a substance associated with a living organism from sedimentation of gold fine particles or sequences.

In all of the above-described techniques, the surface where a physiologically active substance is immobilized is important. Surface plasmon resonance (SPR), which is most commonly used in this technical field, will be described below as an example.

A commonly used measurement chip comprises a transparent substrate (e.g., glass), an evaporated metal film, and a thin film having thereon a functional group capable of immobilizing a physiologically active substance. The measurement chip immobilizes the physiologically active substance on the metal surface via the functional group. A specific binding reaction between the physiological active substance and a test substance is measured, so as to analyze an interaction between biomolecules.

With regard to a thin film having a functional group capable of immobilizing a physiologically active substance, Japanese Patent No. 2815120 discloses a measurement chip

As a thin film having a functional group capable of immobilizing a physiologically active substance, there has been reported a measurement chip where a physiologically active substance is immobilized by using a functional group binding to metal, a linker with a chain length of 10 or more atoms, and a compound having a functional group capable of binding to the physiologically active substance (Japanese Patent No. 2815120). Moreover, a measurement chip comprising a metal film and a plasma-polymerized film formed on the metal film has been reported (Japanese Patent Laid-Open No. 9-264843).

A biosensor that rapidly detects or assays substances that interact with physiologically active substances (usually proteins) immobilized on its sensor surface is required to be able to immobilize as many physiologically active substances as possible since the amounts of the immobilized physiologically active substances directly influence sensor sensitivity. In the case of a surface plasmon resonance biosensor, physiologically active substances are immobilized on the sensor surface by bringing the pH level to a level not lower than the isoelectric point of carboxylic acid and to a level not higher than that of the physiologically active substances with the use of carboxymethylated dextran, so as to electrostatically guide the physiologically active substances to the surface. In the case of a surface plasmon resonance biosensor, however, the binding signals of the substances that bind to the physiologically active substances depend on the molecular weights of such substances. Accordingly, the ability to immobilize larger quantities of physiologically active substances is desired, in order to assay the binding of low-molecular-weight compounds.

Further, on a biosensor that rapidly detects or assays substances that interact with physiologically active substances (usually proteins) immobilized on its sensor surface, physiologically active substances gradually become deactivated, in general. This requires the preparation of physiologically active substances that are immobilized on different surfaces every given period of time. Thus, a method for more rapidly preparing a surface capable of immobilizing physiologically active substances is desired. In the case of a surface plasmon resonance biosensor, physiologically active substances are immobilized on the sensor surface by bringing the pH level to a level not lower than the isoelectric point of carboxylic acid and to a level not higher than that of the physiologically active substances with the use of carboxymethylated dextran, so as to electrostatically guide the physiologically active substances to the surface. Immobilization of physiologically active substances, however, requires strong bonds, such as covalent bonds, between the guided physiologically active substances and reactive groups on the surface. In this respect, dextran is not always a useful material. The speed of immobilization can be increased by increasing the concentration of physiologically active substances in a solution at the time of immobilization. Increased concentration, however, results in nonspecific adsorption of physiologically active substances, which in turn results in unexpected negative baseline fluctuation at the time of assay.

### Disclosure of Invention

It is an object of the present invention to solve the aforementioned problem. That is to say, one object of the present invention is to provide a biosensor that is capable of immobilizing large quantities of physiologically active substances thereon. Another object of the present invention is to provide a biosensor capable of rapidly immobilizing physiologically active substances upon itself and exhibiting small baseline fluctuation.

In order to attain the above objects, the present inventors have conducted concentrated studies. As a result, they discovered that the amount of the immobilized physiologically active substances could be increased by using linear polysaccharides as water-soluble polymers on the biosensor surface. This has led to the completion of the present invention. Further, in order to attain the above objects, the present inventors have conducted concentrated studies. As a result, they discovered that the speed of immobilization of physiologically active substances could be increased and that baseline fluctuation could be lowered with the use of a polysaccharide having 0.3 to 2 primary alcohol groups per unit as a water-soluble polymer on the biosensor surface. This has led to the completion of the present invention.

Thus, the present invention provides a biosensor comprising a substrate having on its surface a polysaccharide capable of chemically immobilizing physiologically active substances, wherein the polysaccharide is a linear polysaccharide or a polysaccharide having 0.3 to 2 primary alcohol groups per unit.

Preferably, the linear polysaccharide is cellulose, pullulan, amylose, agarose, chitin, chitosan, carragheenan, pectin, or a derivative of any of such substances.

Preferably, the polysaccharide having 0.3 to 2 primary alcohol groups per unit is cellulose, pullulan, amylose, amylopectin, guar gum, glycogen, agarose, chitin, chitosan, carragheenan, pectin, glucosaminoglucans, or a derivative of any of such substances.

Preferably, the biosensor according to the present invention is used for nonelectrochemical detection, and is more preferably used for surface plasmon resonance analysis.

Preferably, the biosensor according to the present invention is used for a method wherein a substance that interacts with the physiologically active substances is detected or measured by using a flow channel system comprising a cell formed on said substrate in a state where the flow of the liquid has been stopped, after the liquid contained in the above flow channel system has been exchanged.

Another aspect of the present invention provides a method for producing the aforementioned biosensor according to the present invention which comprises a step of bringing a linear polysaccharide or a polysaccharide having 0.3 to 2 primary alcohol groups per unit into contact with a substrate.

Further another aspect of the present invention provides the biosensor according to the present invention, wherein the physiologically active substance is bound to the surface via covalent bond.

Further another aspect of the present invention provides a method for detecting or measuring a substance that interacts with a physiologically active substance, which comprises a step of bringing the biosensor according to the present invention having on its surface a physiologically active substance bound thereto via covalent bond into contact with a test substance.

Preferably, the substance that interacts with a physiologically active substance is detected or measured by a nonelectrochemical method, and is more preferably detected or measured by surface plasmon resonance analysis.

### BRIEF DESCRIPTION OF THE DRAWINGS

Figure 1 shows a surface plasmon resonance measurement device.
Figure 2 shows a dielectric block.

In figures, 10 indicates measurement unit, 11 indicates dielectric block, 12 indicates metal film, 13 indicates sample-retaining frame, 14 indicates sensing substance, 31 indicates laser light source, 32 indicates condenser lens, 40 indicates light detector, S40 indicates output signal, 400 indicates guide rod, 401 indicates slide block, 402 indicates precision screw, 403 indicates pulse motor, 404 indicates motor controller, 410 indicates unit connector, and 411 indicates connecting member.

### BEST MODE FOR CARRYING OUT THE INVENTION

The embodiments of the present invention will be described below.

The biosensor of the present invention comprises a substrate having on its surface polysaccharides capable of chemically immobilizing physiologically active substances, wherein the polysaccharide is a linear polysaccharide or a polysaccharide having 0.3 to 2 primary alcohol groups per unit. More specifically, the substrate of the biosensor of the present invention is coated with a linear polysaccharide or a polysaccharide having 0.3 to 2 primary alcohol groups per unit.

The term "polysaccharides" refers to polymer compounds, wherein monosaccharides or derivatives thereof are bound to each other via glycosidic bonds. The glycosidic bond sites lie between positions 1 and 4, and between positions 1 and 6 of monosaccharides (mainly, glucose). Specifically, bonds can be extended toward three directions, i.e., toward positions 1, 4, and 6 from a single monosaccharide. A polysaccharide, wherein all monosaccharides except for terminuses are bidirectionally bound, is referred to as a "linear polysaccharide," and a polysaccharide, wherein some monosaccharides are branched due to tridirectional bond, is referred to as a "branched polysaccharide."

Examples of linear polysaccharides that can be used in the present invention include cellulose, pullulan, amylose, agarose, chitin, chitosan, carragheenan, pectin, and a derivative of any of such substances. Cellulose, pullulan, amylose, agarose, and a derivative of any of such substances are particularly preferable. For example, the term "derivative" used herein refers to a polysaccharide in which part of a hydroxyl group in the unit is alkylated, hydroxyalkylated, or the like. Examples thereof include hydroxyethylcellulose, methylcellulose, ethylcellulose, hydroxyethylcellulose, hydroxyethylmethylcellulose, hydroxypropylcellulose, and hydroxypropyl methylcellulose.

The term "polysaccharides having 0.3 to 2 primary alcohol groups per unit" refers to the fact that the number of primary alcohol groups which the polysaccharide has per unit is between 0.3 and 2. A polysaccharide preferably has 0.5 to 2, and more preferably 0.7 to 2, primary alcohol groups per unit.

The glycosidic bond sites may lie between positions 1 and 4, between positions 1 and 3, and between positions 1 and 6 of monosaccharides (mainly glucose). Since a hydroxyl group at position 6 of glucose is a primary alcohol group and that at position 1 or 4 is a secondary alcohol group, dextran comprising 1,6-glycosidic bond has few primary alcohol groups. When a monosaccharide constituting a polysaccharide molecule is determined to be a unit, the number of primary alcohol groups per dextran unit is 0.2 or smaller.

Requirements for a primary alcohol group are: (1) -CH₂OH at position 6 remains (i.e., 1,4-bond or 1,3-bond mainly takes place); and (2) -OH at position 3 or 4 in the unit is converted into -O-L-OH (L generally represents alkylene (-(CH₂)ₙ-)). A specific example of (1) is callose (1,3-bond). Specific examples of (2) include hydroxyethyl dextran and hydroxypropyl dextran. Examples of polysaccharides that satisfy both requirements (1) and (2) and have 2 primary alcohol groups per unit include hydroxyethylcellulose and hydroxypropylcellulose.

Examples of polysaccharides having 1,4-glycosidic bond and 0.3 to 2 primary alcohol groups per unit include cellulose, pullulan, amylose, amylopectin, guar gum, glycogen, agarose, chitin, chitosan, carragheenan, pectin, glucosaminoglucans, and a derivative of any of such substances. Cellulose, pullulan, amylose, amylopectin, agarose, and a derivative of any of such substances are particularly preferable. The term "derivative" used herein refers to a polysaccharide, wherein part of a hydroxyl group in the unit is alkylated, hydroxyalkylated, or the like. Examples thereof include hydroxyethylcellulose, methylcellulose, ethylcellulose, hydroxyethylcellulose, hydroxyethylmethylcellulose, hydroxypropylcellulose, and hydroxypropyl methylcellulose.

The aforementioned linear polysaccharide or polysaccharide having 0.3 to 2 primary alcohol groups per unit may be immobilized on a substrate via a self-assembled monolayer or hydrophobic polymer compound as described hereinafter. In the present invention, specifically, a substrate may be first provided with a self-assembled monolayer or coated with a hydrophobic polymer, and then coated with a linear polysaccharide or a polysaccharide having 0.3 to 2 primary alcohol groups per unit. Hereafter, a self-assembled monolayer and a hydrophobic polymer compound are described.

The term "self-assembled monolayer" used herein refers to a ultrathin membrane, such as a monomolecular membrane or LB membrane, which is formed by the mechanism of its constituting material and which has a given organized structure without the application of external restrictive control. This self-assembling results in an organized structure or pattern over a long distance under nonequilibrium conditions.

For example, a self-assembled monolayer can be prepared from a sulfur-containing compound. Preparation of a self-assembled monolayer on a gold surface with a sulfur-containing compound is described in, for example, Nuzzo, R.G. et al., 1983, J Am Chem Soc, vol. 105, pp. 4481 to 4483; Porter, MD. et al., 1987, J Am Chem Soc, vol. 109, pp. 3559 to 3568; and Troughton, E.B. et al., 1988, Langmuir, vol. 4, pp. 365 to 385.

A sulfur-containing compound is preferably represented by a formula: X-R-Y.

X represents a group capable of binding to a metal membrane. Specifically, asymmetric or symmetric sulfide (-SSR'Y", -SSRY), sulfide (-SR'Y", -SRY), diselenide (-SeSeR'Y", -SeSeRY), selenide (SeR'Y", -SeRY), thiol (-SH), nitrile (-CN), isonitrile, nitro (-NO₂), selenol (-SeH), a trivalent phosphorus compound, isothiocyanate, xanthate, thiocarbamate, phosphine, thioacid, or dithioacid (-COSH, -CSSH) is preferably used.

R (or R') is occasionally interrupted by a hetero atom. It is preferably linear (not branched) because of adequately dense packing, and it is a hydrocarbon chain occasionally containing double and/or triple bonds. The chain usually comprises 5 or more atoms, preferably 10 or more atoms, and further preferably 10 to 30 atoms. A carbon chain can be perfluorinated, according to need. In the case of an asymmetric molecule, R' or R may be H.

Y and Y" are groups that bind to hydrophilic compounds. Y and Y" are preferably identical to each other, which can directly bind to a hydrophilic compound (e.g., hydrogel) or bind thereto following activation. Specific examples thereof that can be used include hydroxyl, carboxyl, amino, aldehyde, hydrazide, carbonyl, epoxy, and vinyl groups.

A compound represented by a formula: X-R-Y, which is in the form of a densely packed monolayer, can bind to a metal surface upon binding of a group represented by X to metal.

Specific examples of a compound represented by a formula: X-R-Y include 10-carboxy-1-decanethiol, 4,4'-dithio dibutyric acid, 11-hydroxy-1-undecanethiol, 11-amino-1-undecanethiol, and 16-hydroxy-1-hexadecanethiol.

A hydrophobic polymer used in the present invention is a polymer having no water-absorbing properties. Its solubility in water (25°C) is 10% or less, more preferably 1% or less, and most preferably 0.1% or less.

A hydrophobic monomer which forms a hydrophobic polymer can be selected from vinyl esters, acrylic esters, methacrylic esters, olefins, styrenes, crotonic esters, itaconic diesters, maleic diesters, fumaric diesters, allyl compounds, vinyl ethers, vinyl ketones, or the like. The hydrophobic polymer may be either a homopolymer consisting of one type of monomer, or copolymer consisting of two or more types of monomers.

Examples of a hydrophobic polymer that is preferably used in the present invention may include polystyrene, polyethylene, polypropylene, polyethylene terephthalate, polyvinyl chloride, polymethyl methacrylate, polyester, and nylon.

A substrate is coated with a hydrophobic polymer according to common methods. Examples of such a coating method may include spin coating, air knife coating, bar coating, blade coating, slide coating, curtain coating, spray method, evaporation method, cast method, and dip method.

The coating thickness of a hydrophobic polymer is not particularly limited, but it is preferably between 0.1 nm and 500 nm, and particularly preferably between 1 nm and 300 nm.

The linear polysaccharide or the polysaccharide having 0.3 to 2 primary alcohol groups per unit which is used in the present invention are capable of chemically immobilizing physiologically active substances. Such polysaccharides preferably comprise functional groups to which physiologically active substances are bound.

Examples of a functional group to which physiologically active substances are bound may include -COOH, -NR¹R² (wherein each of R¹ and R² independently represents a hydrogen atom or lower alkyl group), -OH, -SH, -CHO, -NR³R¹R² (wherein each of R¹, R² and R³ independently represents a hydrogen atom or lower alkyl group), -NCO, -NCS, an epoxy group, and a vinyl group. The number of carbon atoms contained in the lower alkyl group is not particularly limited herein. However, it is generally about C1 to C10, and preferably C1 to C6.

The functional group to which physiologically active substances is preferably a carboxyl group, an amino group, or a hydroxyl group.

In the present invention, the functional group to which physiologically active substances are bound is selected in accordance with a method for immobilizing physiologically active substances. Specifically, the functional group of a given type (e.g., hydroxyl groups) may or may not be "the functional group to which physiologically active substances are bound" depending on a method for immobilizing physiologically active substances.

When the functional group to which physiologically active substances are bound is carboxyl group, for example, carbodiimide is often used in combination with N-hydroxysuccini.mide to generate active ester and further form covalent bonds of active esters with amino groups of physiologically active substances. In such a case, functional groups incapable of binding physiologically active substances, such as hydroxyl groups, amino groups, or polyether compounds, are introduced onto a surface that is not provided with functional groups to which physiologically active substances are bound.

When the functional group to which physiologically active substances are bound is amino group, glutaraldehyde is often allowed to act thereon, and the functional group is allowed to form covalent bonds with amino groups of physiologically active substances. Also, physiologically active substances are oxidized with periodate, and are allowed to directly form covalent bonds with amino groups. In such a case, functional groups incapable of binding physiologically active substances, such as hydroxyl groups, carboxyl groups, or polyether compounds, are introduced onto a surface that is not provided with functional groups to which physiologically active substances are bound.

When the functional group to which physiologically active substances are bound is hydroxyl group, polyepoxy compounds or epichlorohydrins are often allowed to act thereon, and the functional group is allowed to form covalent bonds with amino groups of physiologically active substances. Chemical bond such as direct ether bond involving the use of a halogenated alkyl may be available. When chemical bond is applied to physiologically active substances, however, it may be difficult to maintain physiological activity. In such a case, functional groups incapable of binding physiologically active substances, such as water-soluble groups (e.g., polyether of polyethylene glycol) containing no labile hydrogen atom (specifically, a hydrogen atom of a hydroxyl, amino, or carboxyl group), may be introduced onto a surface that is not provided with functional groups to which physiologically active substances are bound.

The biosensor of the present invention has as broad a meaning as possible, and the term biosensor is used herein to mean a sensor, which converts an interaction between biomolecules into a signal such as an electric signal, so as to measure or detect a target substance. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance, as described above, is immobilized on a substrate to be used as a molecule-recognizing substance, so that the corresponding substance can be selectively measured.

In the biosensor of the present invention, a metal surface or metal film is coated with a hydrophilic compound. A metal constituting the metal surface or metal film is not particularly limited, as long as surface plasmon resonance is generated when the metal is used for a surface plasmon resonance biosensor. Examples of a preferred metal may include free-electron metals such as gold, silver, copper, aluminum or platinum. Of these, gold is particularly preferable. These metals can be used singly or in combination. Moreover, considering adherability to the above substrate, an interstitial layer consisting of chrome or the like may be provided between the substrate and a metal layer.

The film thickness of a metal film is not limited. When the metal film is used for a surface plasmon resonance biosensor, the thickness is preferably between 0.1 nm and 500 nm, more preferably between 0.5 nm and 500 nm, and particularly preferably between 1 nm and 200 nm. If the thickness exceeds 500 nm, the surface plasmon phenomenon of a medium cannot be sufficiently detected. Moreover, when an interstitial layer consisting of chrome or the like is provided, the thickness of the interstitial layer is preferably between 0.1 nm and 10 nm.

Formation of a metal film may be carried out by common methods, and examples of such a method may include sputtering method, evaporation method, ion plating method, electroplating method, and nonelectrolytic plating method.

A metal film is preferably placed on a substrate. The description "placed on a substrate" is used herein to mean a case where a metal film is placed on a substrate such that it directly comes into contact with the substrate, as well as a case where a metal film is placed via another layer without directly coming into contact with the substrate. When a substrate used in the present invention is used for a surface plasmon resonance biosensor, examples of such a substrate may include, generally, optical glasses such as BK7, and synthetic resins. More specifically, materials transparent to laser beams, such as polymethyl methacrylate, polyethylene terephthalate, polycarbonate or a cycloolefin polymer, can be used. For such a substrate, materials that are not anisotropic with regard to polarized light and have excellent workability are preferably used.

The biosensor of the present invention preferably has a functional group capable of immobilizing a physiologically active substance on the outermost surface of the substrate. The term "the outermost surface of the substrate" is used to mean "the surface, which is farthest from the substrate," and more specifically, it means "the surface of a compound applied on a substrate, which is farthest from the substrate."

In the biosensor surface obtained as mentioned above, a physiologically active substance is covalently bound thereto via the above functional group, so that the physiologically active substance can be immobilized on the metal surface or the metal film.

A physiologically active substance immobilized on the surface for the biosensor of the present invention is not particularly limited, as long as it interacts with a measurement target. Examples of such a substance may include an immune protein, an enzyme, a microorganism, nucleic acid, a low molecular weight organic compound, a nonimmune protein, an immunoglobulin-binding protein, a sugar-binding protein, a sugar chain recognizing sugar, fatty acid or fatty acid ester, and polypeptide or oligopeptide having a ligand-binding ability.

Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, methicillin-resistant *Staphylococcus aureus*, antibiotic, narcotic drug, cocaine, heroin, crack or the like, there can be used, for example, an anti-atrazine antibody, anti-kanamycin antibody, anti-metamphetamine antibody, or antibodies against O antigens 26, 86, 55, 111 and 157 among enteropathogenic *Escherichia coli*.

An enzyme used as a physiologically active substance herein is not particularly limited, as long as it exhibits an activity to a measurement target or substance metabolized from the measurement target. Various enzymes such as oxidoreductase, hydrolase, isomerase, lyase or synthetase can be used. More specifically, when a measurement target is glucose, glucose oxidase is used, and when a measurement target is cholesterol, cholesterol oxidase is used. Moreover, when a measurement target is an agricultural chemical, pesticide, methicillin-resistant *Staphylococcus aureus*, antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.

A microorganism used as a physiologically active substance herein is not particularly limited, and various microorganisms such as *Escherichia coli* can be used.

As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.

As a low molecular weight organic compound, any given compound that can be synthesized by a common method of synthesizing an organic compound can be used.

A nonimmune protein used herein is not particularly limited, and examples of such a nonimmune protein may include avidin (streptoavidin), biotin, and a receptor.

Examples of an immunoglobulin-binding protein used herein may include protein A, protein G, and a rheumatoid factor (RF).

As a sugar-binding protein, for example, lectin is used.

Examples of fatty acid or fatty acid ester may include stearic acid, arachidic acid, behenic acid, ethyl stearate, ethyl arachidate, and ethyl behenate.

When a physiologically active substance is a protein such as an antibody or enzyme, or nucleic acid, an amino group, thiol group or the like of the physiologically active substance is covalently bound to a functional group located on a metal surface, so that the physiologically active substance can be immobilized on the metal surface.

A biosensor to which a physiologically active substance is immobilized as described above can be used to detect and/or measure a substance which interacts with the physiologically active substance.

Namely, the present invention provides a method for detecting and/or measuring a substance that interacts with a physiologically active substance, which comprises a step of bringing the biosensor according to the present invention having on its surface a physiologically active substance bound thereto into contact with a test substance.

As a test substance, a sample containing a substance interacting with the aforementioned physiologically active substance can be used, for example.

In the present invention, it is preferable to detect and/or measure an interaction between a physiologically active substance immobilized on the surface used for a biosensor and a test substance by a nonelectric chemical method. Examples of a non-electrochemical method may include a surface plasmon resonance (SPR) measurement technique, a quartz crystal microbalance (QCM) measurement technique, and a measurement technique that uses functional surfaces ranging from gold colloid particles to ultra-fine particles.

In a preferred embodiment of the present invention, the biosensor of the present invention can be used as a biosensor for surface plasmon resonance which is characterized in that it comprises a metal film placed on a transparent substrate.

A biosensor for surface plasmon resonance is a biosensor used for a surface plasmon resonance biosensor, meaning a member comprising a portion for transmitting and reflecting light emitted from the sensor and a portion for immobilizing a physiologically active substance. It may be fixed to the main body of the sensor or may be detachable.

The surface plasmon resonance phenomenon occurs due to the fact that the intensity of monochromatic light reflected from the border between an optically transparent substance such as glass and a metal thin film layer depends on the refractive index of a sample located on the outgoing side of the metal. Accordingly, the sample can be analyzed by measuring the intensity of reflected monochromatic light.

A device using a system known as the Kretschmann configuration is an example of a surface plasmon measurement device for analyzing the properties of a substance to be measured using a phenomenon whereby a surface plasmon is excited with a lightwave (for example, Japanese Patent Laid-Open No. 6-167443). The surface plasmon measurement device using the above system basically comprises a dielectric block formed in a prism state, a metal film that is formed on a face of the dielectric block and comes into contact with a measured substance such as a sample solution, a light source for generating a light beam, an optical system for allowing the above light beam to enter the dielectric block at various angles so that total reflection conditions can be obtained at the interface between the dielectric block and the metal film, and a light-detecting means for detecting the state of surface plasmon resonance, that is, the state of attenuated total reflection, by measuring the intensity of the light beam totally reflected at the above interface.

The biosensor of the present invention can preferably be formed in a measurement chip used for a surface plasmon resonance measurement device comprising a dielectric block, a metal film formed on one side of the dielectric block, a light source for generating a light beam, an optical system for allowing the above light beam to enter the above dielectric block so that total reflection conditions can be obtained at the interface between the dielectric block and the metal film and so that various incidence angles can be included, and a light-detecting means for detecting the state of surface plasmon resonance by measuring the intensity of the light beam totally reflected at the above interface. The aforementioned measurement chip is basically composed of the above dielectric block and the above metal film, wherein the above dielectric block is formed as a block including all of an incidence face and an exit face for the above light beam and a face on which the above metal film is formed, and wherein the above metal film is unified with this dielectric block.

In order to achieve various incident angles as described above, a relatively thin light beam may be caused to enter the above interface while changing an incident angle. Otherwise, a relatively thick light beam may be caused to enter the above interface in a state of convergent light or divergent light, so that the light beam contains components that have entered therein at various angles. In the former case, the light beam whose reflection angle changes depending on the change of the incident angle of the entered light beam can be detected with a small photodetector moving in synchronization with the change of the above reflection angle, or it can also be detected with an area sensor extending along the direction in which the reflection angle is changed. In the latter case, the light beam can be detected with an area sensor extending to a direction capable of receiving all the light beams reflected at various reflection angles.

With regard to a surface plasmon measurement device with the above structure, if a light beam is allowed to enter the metal film at a specific incident angle greater than or equal to a total reflection angle, then an evanescent wave having an electric distribution appears in a measured substance that is in contact with the metal film, and a surface plasmon is excited by this evanescent wave at the interface between the metal film and the measured substance. When the wave vector of the evanescent light is the same as that of a surface plasmon and thus their wave numbers match, they are in a resonance state, and light energy transfers to the surface plasmon. Accordingly, the intensity of totally reflected light is sharply decreased at the interface between the dielectric block and the metal film. This decrease in light intensity is generally detected as a dark line by the above light-detecting means. The above resonance takes place only when the incident beam is p-polarized light. Accordingly, it is necessary to set the light beam in advance such that it enters as p-polarized light.

If the wave number of a surface plasmon is determined from an incident angle causing the attenuated total reflection (ATR), that is, an attenuated total reflection angle (θSP), the dielectric constant of a measured substance can be determined. As described in Japanese Patent Laid-Open No. 11-326194, a light-detecting means in the form of an array is considered to be used for the above type of surface plasmon measurement device in order to measure the attenuated total reflection angle (θSP) with high precision and in a large dynamic range. This light-detecting means comprises multiple photo acceptance units that are arranged in a certain direction, that is, a direction in which different photo acceptance units receive the components of light beams that are totally reflected at various reflection angles at the above interface.

In the above case, there is established a differentiating means for differentiating a photodetection signal outputted from each photo acceptance unit in the above array-form light-detecting means with regard to the direction in which the photo acceptance unit is arranged. An attenuated total reflection angle (θSP) is then specified based on the derivative value outputted from the differentiating means, so that properties associated with the refractive index of a measured substance are determined in many cases.

In addition, a leaking mode measurement device described in "Bunko Kenkyu (Spectral Studies)" Vol. 47, No. 1 (1998), pp. 21 to 23 and 26 to 27 has also been known as an example of measurement devices similar to the above-described device using attenuated total reflection (ATR). This leaking mode measurement device basically comprises a dielectric block formed in a prism state, a clad layer that is formed on a face of the dielectric block, a light wave guide layer that is formed on the clad layer and comes into contact with a sample solution, a light source for generating a light beam, an optical system for allowing the above light beam to enter the dielectric block at various angles so that total reflection conditions can be obtained at the interface between the dielectric block and the clad layer, and a light-detecting means for detecting the excitation state of waveguide mode, that is, the state of attenuated total reflection, by measuring the intensity of the light beam totally reflected at the above interface.

In the leaking mode measurement device with the above structure, if a light beam is caused to enter the clad layer via the dielectric block at an incident angle greater than or equal to a total reflection angle, only light having a specific wave number that has entered at a specific incident angle is transmitted in a waveguide mode into the light wave guide layer, after the light beam has penetrated the clad layer. Thus, when the waveguide mode is excited, almost all forms of incident light are taken into the light wave guide layer, and thereby the state of attenuated total reflection occurs, in which the intensity of the totally reflected light is sharply decreased at the above interface. Since the wave number of a waveguide light depends on the refractive index of a measured substance placed on the light wave guide layer, the refractive index of the measurement substance or the properties of the measured substance associated therewith can be analyzed by determining the above specific incident angle causing the attenuated total reflection.

In this leaking mode measurement device also, the above-described array-form light-detecting means can be used to detect the position of a dark line generated in a reflected light due to attenuated total reflection. In addition, the above-described differentiating means can also be applied in combination with the above means.

The above-described surface plasmon measurement device or leaking mode measurement device may be used in random screening to discover a specific substance binding to a desired sensing substance in the field of research for development of new drugs or the like. In this case, a sensing substance is immobilized as the above-described measured substance on the above thin film layer (which is a metal film in the case of a surface plasmon measurement device, and is a clad layer and a light guide wave layer in the case of a leaking mode measurement device), and a sample solution obtained by dissolving various types of test substance in a solvent is added to the sensing substance. Thereafter, the above-described attenuated total reflection angle (θSP) is measured periodically when a certain period of time has elapsed.

If the test substance contained in the sample solution is bound to the sensing substance, the refractive index of the sensing substance is changed by this binding over time. Accordingly, the above attenuated total reflection angle (θSP) is measured periodically after the elapse of a certain time, and it is determined whether or not a change has occurred in the above attenuated total reflection angle (θSP), so that a binding state between the test substance and the sensing substance is measured. Based on the results, it can be determined whether or not the test substance is a specific substance binding to the sensing substance. Examples of such a combination between a specific substance and a sensing substance may include an antigen and an antibody, and an antibody and an antibody. More specifically, a rabbit anti-human IgG antibody is immobilized as a sensing substance on the surface of a thin film layer, and a human IgG antibody is used as a specific substance.

It is to be noted that in order to measure a binding state between a test substance and a sensing substance, it is not always necessary to detect the angle itself of an attenuated total reflection angle (θSP). For example, a sample solution may be added to a sensing substance, and the amount of an attenuated total reflection angle (θSP) changed thereby may be measured, so that the binding state can be measured based on the magnitude by which the angle has changed. When the above-described array-form light-detecting means and differentiating means are applied to a measurement device using attenuated total reflection, the amount by which a derivative value has changed reflects the amount by which the attenuated total reflection angle (θSP) has changed. Accordingly, based on the amount by which the derivative value has changed, a binding state between a sensing substance and a test substance can be measured (Japanese Patent Application No. 2000-398309 filed by the present applicant). In a measuring method and a measurement device using such attenuated total reflection, a sample solution consisting of a solvent and a test substance is added dropwise to a cup- or petri dish-shaped measurement chip wherein a sensing substance is immobilized on a thin film layer previously formed at the bottom, and then, the above-described amount by which an attenuated total reflection angle (θSP) has changed is measured.

Moreover, Japanese Patent Laid-Open No. 2001-330560 describes a measurement device using attenuated total reflection, which involves successively measuring multiple measurement chips mounted on a turntable or the like, so as to measure many samples in a short time.

When the biosensor of the present invention is used in surface plasmon resonance analysis, it can be applied as a part of various surface plasmon measurement devices described above.

Further, the biosensor according to the present invention can be used for a method wherein a substance that interacts with the physiologically active substances is detected or measured by using a flow channel system comprising a cell formed on the substrate in a state where the flow of the liquid has been stopped, after the liquid contained in the above flow channel system has been exchanged. For example, by using a surface plasmon resonance measurement device comprising a flow channel system having a cell formed on a metal film and a light-detecting means for detecting the state of surface plasmon resonance by measuring the intensity of a light beam totally reflected on the meal film, and exchanging the liquid contained in the above flow channel system, a change in surface plasmon resonance can be measured in a state where the flow of the liquid has been stopped, after the liquid contained in the above flow channel system has been exchanged.

The time of the stop of the flow of the liquid is not particularly limited. For example, it may be between 1 second and 30 minutes, preferably between 10 seconds and 20 minutes, and more preferably between 1 minute and 20 minutes.

Preferably, the liquid contained in a flow channel system is exchanged from a reference liquid containing no test substance to be measured to a sample liquid containing a test substance to be measured, and thereafter, a change in surface plasmon resonance can be measured in a state where the flow of the sample liquid has been stopped.

Preferably, a reference cell, to which a substance interacting with a test substance does not bind, is connected in series with a detection cell, to which a substance interacting with a test substance binds, the connected cells are placed in a flow channel system, and a liquid is then fed through the reference cell and the detection cell, so that a change in surface plasmon resonance can be measured.

In addition, the ratio (Ve/Vs) of the amount of a liquid exchanged (Ve ml) in a single measurement to the volume (Vs ml) of a cell used in measurement (and when the aforementioned reference cell and detection cell are used, the total volume of these cells) is preferably between 1 and 100. Ve/Vs is more preferably between 1 and 50, and particularly preferably between 1 and 20. The volume (Vs ml) of a cell used in measurement is not particularly limited. It is preferably between 1 x 10⁻⁶ and 1.0 ml, and particularly preferably between 1 x 10⁻⁵ and 1 x 10⁻¹ ml. The period of time necessary for exchanging the liquid is preferably between 0.01 second and 100 seconds, more preferably between 0.1 second and 10 seconds.

The present invention will be further specifically described in the following examples. However, the examples are not intended to limit the scope of the present invention.

### EXAMPLES

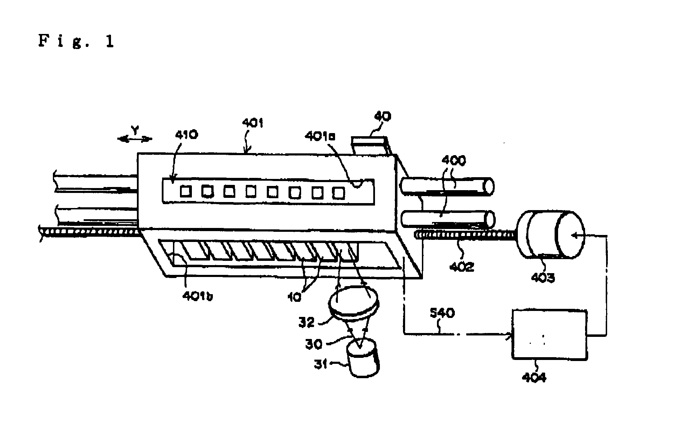

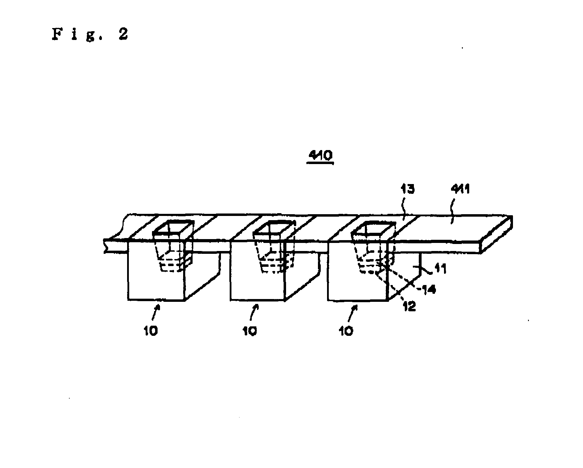

The following experiment was carried out using a device shown in Figure 22 of Japanese Patent Laid-Open No. 2001-330560 (hereinafter referred to as the surface plasmon resonance measurement device of the present invention) (shown in Figure 1 of the present specification) and a dielectric block shown in Figure 23 of Japanese Patent Laid-Open No. 2001-330560 (hereinafter referred to as the dielectric block of the present invention) (shown in Figure 2 of the present specification).

In the surface plasmon resonance measurement device shown in Figure 1, a slide block 401 is used as a supporting medium for supporting measurement units, which is joined to two guide rods 400, 400 placed in parallel with each other while flexibly sliding in contact, and which also flexibly moves linearly along the two rods in the direction of an arrow Y in the figure. The slide block 401 is screwed together with a precision screw 402 placed in parallel with the above guide rods 400, 400, and the precision screw 402 is reciprocally rotated by a pulse motor 403 which constitutes a supporting medium-driving means together with the precision screw 402.

It is to be noted that the movement of the pulse motor 403 is controlled by a motor controller 404. This is to say, an output signal S 40 of a linear encoder (not shown in the figure), which is incorporated into the slide block 401 and detects the position of the slide block 401 in the longitudinal direction of the guide rods 400, 400, is inputted into the motor controller 404. The motor controller 404 controls the movement of the pulse motor 403 based on the signal S 40.

Moreover, below the guide rods 400, 400, there are established a laser light source 31 and a condenser 32 such that they sandwich from both sides the slide block 401 moving along the guide rods, and a photodetector 40. The condenser 32 condenses a light beam 30. In addition, the photodetector 40 is placed thereon.

In this embodiment, a stick-form unit connected body 410 obtained by connecting and fixing eight measurement units 10 is used as an example, and the measurement units 10 are mounted on the slide block 401 in a state in which eight units are arranged in a line.

Figure 2 shows the structure of the unit connected body 410 in detail. As shown in the figure, the unit connected body 410 is obtained by connecting the eight measurement units 10 by a connecting member 411.

This measurement unit 10 is obtained by molding a dielectric block 11 and a sample-retaining frame 13 into one piece, for example, using transparent resin or the like. The measurement unit constitutes a measurement chip that is exchangeable with respect to a turntable. In order to make the measurement chip exchangeable, for example, the measurement unit 10 may be fitted into a through-hole that is formed in the turntable. In the present example, a sensing substance 14 is immobilized on a metal film 12.

### Example 1:

### (1) Preparation of measurement chip

The dielectric block of the present invention, onto which 50 nm gold has been vapor-deposited as a metal membrane, was treated with the UV-Ozone Cleaning System (Model-208; Technovision, Inc.) for 30 minutes. Then, 5.0 mM solution of 11-hydroxy-1-undecanethiol in ethanol/water (80/20) was added so as to be in contact with a metal membrane, and surface treatment was carried out at 25°C for 18 hours. Thereafter, the block was washed five times with ethanol, once with an ethanol-water mixed solvent, and 5 times with water.

Subsequently, the 11-hydroxy-1-undecanethiol-coated surface was brought into contact with a solution containing 10% by weight of epichlorohydrin (solvent: 1:1 mixed solution of 0.4M sodium hydroxide and diethylene glycol dimethyl ether), and the reaction was allowed to proceed in a shake incubator (25°C) for 4 hours. The surface was washed twice with ethanol and five times with water.

Subsequently, 4.5 ml of 1M sodium hydroxide was added to 40.5 ml of an aqueous solution of 25% by weight of polysaccharide shown in Table 1, and the resulting solution was brought into contact with the epichlorohydrin-treated surface. Incubation was then carried out in a shake incubator at 25°C for 20 hours. The surface was washed 10 times with water at 50°C.

Subsequently, 3.5 g of bromoacetic acid was dissolved in 27g of 2M sodium hydroxide solution, and the resultant was brought into contact with the dextran-treated surface, and incubation was carried out in a shake incubator at 35°C for 3 hours. The surface was washed with water, and the aforementioned procedure was then repeated three times.

### (2) Preparation of protein-immobilized chip

The solution in the measurement chip was removed, and the dielectric block was covered with silicone rubber to prepare a cell having an internal volume of 15 µl. The silicone rubber cover was provided with two pores with diameters of 1 mm, and a teflon tube (i.d.: 0.5 mm; o.d.: 1 mm) was allowed to path therethrough to prepare a flow channel system. The chip provided with such flow channel system was mounted on the surface plasmon resonance measurement device of the present invention. Hereafter, liquid exchange was carried out by pouring 500 µl of liquid for 5 seconds.

The flow channel was filled with 70 µl of a mixed solution of 200 mM EDC (N-ethyl-N'-(3-dimethylaminopropyl)carbodiimide hydrochloride) and 50 mM NHS (N-hydroxysuceinimide), and was then allowed to stand for 10 minutes. After the liquid exchange was carried out twice with 100 µl of buffer shown in Table 1 (BIAcore), the liquid was exchanged with the acetate buffer containing a protein with the concentration and pH shown in Table 1, and the resultant was allowed to stand for 30 minutes to immobilize proteins. The inside of the chip was exchanged with 1 M ethanolamine solution and was then allowed to stand for 10 minutes. The interior of the chip was exchanged twice with 100 µl of buffer shown in Table 1 to prepare a protein-immobilized chip. The amount of the immobilized proteins was determined based on changes in signal intensities before and after protein immobilization. The results are shown in Table 1.

### (3) Evaluation of binding ability of hydrochlorothiazide

The flow channel system of the CA-immobilized chips, the amount of CA immobilized thereto was approximately 3,000 RU, was filled with 1x PBS buffer (pH 7.4). Based on the signal intensity before liquid exchange, changes in the signal intensity were measured at intervals of 0.5 seconds. The content of the flow channel system was exchanged with a hydrochlorothiazide solution (Sigma) (dissolved in 1× PBS (pH 7.4) to result in 50 µM therein). Changes in signal intensities were determined from 5 seconds before the initiation of the exchange to 2 minutes after the exchange.

**Table 1**

| Level | Polysaccharide | | Acetate | Protein | Protein concentration | Amount of protein immobilized | Hydrochloro- Thiazide | Note |
|---|---|---|---|---|---|---|---|---|
| | Name | Linear/ branched | pH | | (µg/ml) | (RU) | Binding amount (RU) | |
| 1 | Dextran | Branched | 5.0 | Carbonic anhydrase | 10 | 1500 | 5 | Comp. Ex. |
| 5 | Pullulan | Linear | 5.0 | Carbonic anhydrase | 10 | 2500 | 10 | Ex. |
| 2 | Dextran | Branched | 5.0 | N-Avidin | 20 | 2500 | - | Comp. Ex. |
| 6 | Pullulan | linear | 5.0 | N-Avidin | 20 | 4000 | - | Ex. |
| 3 | Dextran | branched | 5.5 | Trypsin | 10 | 600 | - | Comp. Ex. |
| 7 | Pullulan | linear | 5.5 | Trypsin | 10 | 2000 | - | Ex. |
| 4 | Dextran | branched | 4.5 | protein A | 50 | 1000 | - | Comp. Ex. |
| 8 | Pullulan | linear | 4.5 | protein A | 50 | 1800 | - | Ex. |

| | | | | | | | | |
|---|---|---|---|---|---|---|---|---|
| Comp.Ex.: Comparative Example Ex.: Example of the present invention | | | | | | | | |

As is apparent from the results shown in Table 1, the biosensor of the present invention that uses linear polysaccharides at the time of protein immobilization is capable of immobilizing larger quantities of proteins. Based on level 1 and level 5, more intensified signals can be obtained concerning the binding of physiologically active substances.

### Example 2:

### (1) Preparation of measurement chip

The dielectric block of the present invention, onto which 50 nm gold has been vapor-deposited as a metal membrane, was treated with the UV-Ozone Cleaning System (Model-208; Technovision, Inc.) for 30 minutes. Then, 5.0 mM solution of 11-hydroxy-1-undecanethiol in ethanol/water (80/20) was added so as to be in contact with a metal membrane, and surface treatment was carried out at 25°C for 18 hours. Thereafter, the block was washed five times with ethanol, once with an ethanol-water mixed solvent, and 5 times with water.

Subsequently, the 11-hydroxy-1-undecanethiol-coated surface was brought into contact with a solution containing 10% by weight of epichlorohydrin (solvent: 1:1 mixed solution of 0.4M sodium hydroxide and diethylene glycol dimethyl ether), and the reaction was allowed to proceed in a shake incubator (25°C) for 4 hours. The surface was washed twice with ethanol and five times with water.

Subsequently, 4.5 ml of 1M sodium hydroxide was added to 40.5 ml of an aqueous solution of polysaccharide, the type and concentration of which are shown in Table 2, and the resulting solution was brought into contact with the epichlorohydrin-treated surface. Incubation was then carried out in a shake incubator at 25°C for 20 hours. The surface was washed 10 times with water at 50°C.

Subsequently, 3.5 g of bromoacetic acid was dissolved in 27g of 2M sodium hydroxide solution, and the resultant mixture was brought into contact with the dextran-treated surface, and incubation was carried out in a shake incubator at 28°C for 16 hours. The surface was washed with water, and the aforementioned procedure was then repeated once.

### (2) Preparation of CA-immobilized chip

The solution in the measurement chip was removed, and the dielectric block was covered with silicone rubber to prepare a cell having an internal volume of 15 µl. The silicone rubber cover was provided with two pores with diameters of 1 mm, and a teflon tube (i.d.: 0.5 mm; o.d.: 1 mm) was allowed to path therethrough to prepare a flow channel system. The chip provided with such flow channel system was mounted on the surface plasmon resonance measurement device of the present invention. Hereafter, liquid exchange was carried out by pouring 500 µl of liquid for 5 seconds.

The flow channel was filled with 70 µl of a mixed solution of 200 mM EDC (N-ethyl-N'-(3-dimethylaminopropyl)carbodiimide hydrochloride) and 50 mM NHS (N-hydroxysuccinimide), and was then allowed to stand for 10 minutes. After the liquid exchange was carried out twice with 100 µl of Acetate 5.0 buffer (BIAcore), the liquid was exchanged with the acetate 5.0 solution containing Bovine Carbonic Anhydrase (hereinafter referred to as CA) (SIGMA) with the concentration shown in Table 2, and the resultant was allowed to stand for a certain period to immobilize CA. The inside of the chip was exchanged with 1 M ethanolamine solution and was then allowed to stand for 10 minutes. The interior of the chip was exchanged twice with 100 µl of Acetate 5.0 buffer to prepare CA-immobilized chip. The amount of the immobilized proteins was determined based on changes in signal intensities before and after CA immobilization. Also, the period for adding CA solution is changed, and the minimum period necessary to achieve the immobilized CA amount of 3000 RU or more was determined.

### (3) Evaluation of binding ability of hydrochlorothiazide

The flow channel system of the CA-immobilized chips, the amount of CA immobilized thereto was approximately 3,000 RU, was filled with 1x PBS buffer (pH 7.4). Based on the signal intensity before liquid exchange, changes in the signal intensity were measured at intervals of 0.5 seconds. The content of the flow channel system was exchanged with a hydrochlorothiazide solution (Sigma) (dissolved in 1 × PBS (pH 7.4) to result in 50 µM therein). Changes in signal intensities were determined from 5 seconds before the initiation of the exchange to 2 minutes after the exchange.

The time period required for immobilization is preferably within 15 minutes. In order to attain highly-reliable data, the binding amount of the compound is preferably 5 RU or higher. The results are shown in Table 2.

**Table 2:**

| Level | CA concent-Ration | Polysaccharide | | | Duration of immobilization^{*2} | Hydorochloro-thiazide | Note |
|---|---|---|---|---|---|---|---|
| | (µg/ml) | Name | Primary alcohol content^{*1} | Concentration (% by weight) | (min) | Binding amount (RU) | |
| 1 | 20 | Dextran | up to 0.1 | 15 | -^{*3} | -^{*3} | Comp. Ex. |
| 2 | 200 | Dextran | up to 0.1 | 15 | 5 | -15 | Comp. Ex. |
| 3 | 20 | Dextran | up to 0.1 | 25 | 45 | 2 | Comp. Ex. |
| 4 | 200 | Dextran | up to 0.1 | 25 | 4 | -30 | Comp. Ex. |
| 5 | 20 | Amylose | 1 | 10 | 12 | 17 | Ex. |
| 6 | 200 | Amylose | 1 | 10 | 3 | 11 | Ex. |
| 7 | 20 | Amylose | 1 | 15 | 9 | 15 | Ex. |
| 8 | 200 | Amylose | 1 | 15 | 3 | 10 | Ex. |

| | | | | | | | |
|---|---|---|---|---|---|---|---|
| *1: a primary alcohol content per unit *2: the minimal duration during which the amount of CA immobilization becomes 3,000 RU or higher *3: the amount of immobilization of 3,000 RU or lower Comp.Ex.: Comparative Example Ex.: Example of the present invention | | | | | | | |

As is apparent from the results shown in Table 2, use of the polysaccharide of the present invention having 0.3 or more primary alcohol groups per unit can shorten the duration required for immobilizing CA at lower CA concentrations.

### EFFECTS OF THE INVENTION

The biosensor of the present invention is capable of immobilizing larger quantities of physiologically active substances. The biosensor of the present invention can rapidly immobilize physiologically active substances upon itself, and it exhibits small baseline fluctuation.

## Claims

1. A biosensor comprising a substrate having on its surface a polysaccharide capable of chemically immobilizing physiologically active substances, wherein the polysaccharide is a linear polysaccharide or a polysaccharide having 0.3 to 2 primary alcohol groups per unit.

2. The biosensor according to claim 1, wherein the linear polysaccharide is cellulose, pullulan, amylose, agarose, chitin, chitosan, carragheenan, pectin, or a derivative of any of such substances.

3. The biosensor according to claim 1, wherein the polysaccharide having 0.3 to 2 primary alcohol groups per unit is cellulose, pullulan, amylose, amylopectin, guar gum, glycogen, agarose, chitin, chitosan, carragheenan, pectin, glucosaminoglucans, or a derivative of any of such substances.

4. The biosensor according to claim 1, which is used for nonelectrochemical detection.

5. The biosensor according to claim 1, which is used for surface plasmon resonance analysis.

6. The biosensor according to claim 1, which is used for a method wherein a substance that interacts with the physiologically active substances is detected or measured by using a flow channel system comprising a cell formed on said substrate in a state where the flow of the liquid has been stopped, after the liquid contained in the above flow channel system has been exchanged.

7. A method for producing the biosensor according to claim 1 which comprises a step of bringing a linear polysaccharide or a polysaccharide having 0.3 to 2 primary alcohol groups per unit into contact with a substrate.

8. The biosensor according to claim 1, wherein the physiologically active substance is bound to the surface via covalent bond.

9. A method for detecting or measuring a substance that interacts with a physiologically active substance, which comprises a step of bringing the biosensor according to claim 1 having on its surface a physiologically active substance bound thereto via covalent bond into contact with a test substance.

10. The method according to claim 9, wherein the substance that interacts with a physiologically active substance is detected or measured by a nonelectrochemical method.

11. The method according to claim 9, wherein the substance that interacts with a physiologically active substance is detected or measured by surface plasmon resonance analysis.
